# EUROPÄISCHE PATENTANMELDUNG

(11) **EP 1 797 817 A1**
(43) Veröffentlichungstag der Anmeldung: **20.06.2007**
(21) Anmeldenummer: 05027755.7
(22) Anmeldetag: 19.12.2005
(51) Int. Cl.: A61B 5/00, C12Q 1/00, G01N 33/487

(54) **Sandwichsensor zur Ermittlung einer Analytkonzentration**

(71) Anmelder: F.HOFFMANN-LA ROCHE AG, 4070 Basel (CH); Roche Diagnostics GmbH, 68305 Mannheim (DE)
(72) Erfinder: Bainczyk, Gregor, 68199 Mannheim (DE); Kotzan, Holger, 68526 Ladenburg (DE)
(74) Vertreter: Huhn, Michael

(57) **Zusammenfassung**

Es wird ein implantierbarer Sensor (110) vorgeschlagen, welcher zur Ermittlung einer Konzentration mindestens eines Analyten in einem Medium, insbesondere einem Körpergewebe und/oder einer Körperflüssigkeit, eingesetzt werden kann. Der implantierbare Sensor (110) weist einen Schichtaufbau auf, wobei der Schichtaufbau mindestens ein isolierendes Trägersubstrat (112; 210, 216) und mindestens zwei Elektroden (134, 136; 238, 240, 242) aufweist. Die mindestens zwei Elektroden (134, 136; 238, 240, 242) umfassen mindestens eine Arbeitselektrode (144) und mindestens eine weitere Elektrode (134, 136; 238, 240, 242), insbesondere mindestens eine Gegenelektrode (140) und/oder mindestens eine Referenzelektrode (142). Das mindestens eine isolierende Trägersubstrat (112; 210, 216) umfasst mindestens ein nichtleitendes elektrisches Material. Die Arbeitselektrode (144) und die mindestens eine weitere Elektrode (134, 136; 234, 240, 242) sind in unterschiedlichen Schichtebenen des Schichtaufbaus angeordnet.

## Beschreibung

### Gebiet der Erfindung:

Es wird ein implantierbarer Sensor zur Ermittlung einer Konzentration mindestens eines Analyten in einem Medium, insbesondere einem Körpergewebe und/oder einer Körperflüssigkeit, eine Vorrichtung, welche den implantierbaren Sensor verwendet, eine Verwendung des implantierbaren Sensors und/oder der Vorrichtung sowie ein Verfahren zur Herstellung des implantierbaren Sensors vorgeschlagen. Derartige Sensoren und/oder Vorrichtungen werden insbesondere im Bereich der Medizintechnik eingesetzt, beispielsweise um elektrochemisch eine Konzentration von Blutglucose, Triglyceriden, Laktat oder anderer Analyten zu bestimmen.

### Stand der Technik:

Die Bestimmung der Blutglucosekonzentrationen sowie eine entsprechende Medikation ist für Diabetiker ein essentieller Bestandteil des Tagesablaufs. Dabei muss die Blutglucosekonzentration schnell und einfach mehrmals am Tag (typischerweise zwei bis sieben Mal) bestimmt werden, um gegebenenfalls entsprechende medizinische Maßnahmen ergreifen zu können. In vielen Fällen erfolgt dabei eine Medikation mittels automatischer Systeme, insbesondere so genannte Insulinpumpen.

Um den Tagesablauf des Diabetikers nicht mehr als nötig einzuschränken, werden häufig entsprechend mobile Geräte eingesetzt, welche einfach zu transportieren und zu handhaben sein sollten, so dass eine Messung der Blutglucosekonzentration, beispielsweise am Arbeitsplatz oder auch in der Freizeit, problemlos erfolgen kann. Derzeit sind verschiedene mobile Geräte am Markt, welche teilweise nach unterschiedlichen Messmethoden und unter Verwendung unterschiedlicher Diagnoseverfahren funktionieren. Ein erstes Messverfahren beruht beispielsweise auf einer elektrochemischen Messmethode, wobei eine Blutprobe, welche vom Patienten beispielsweise durch Perforieren einer Hautschicht mittels einer Lanzette dem Körpergewebe entnommen wird, auf eine mit Enzymen und Mediatoren beschichtete Elektrode appliziert wird. Entsprechende Teststreifen für derartige elektrochemische Messverfahren sind beispielsweise in US 5,286,362 beschrieben. Andere bekannte Messmethoden verwenden optische Messverfahren, welche beispielsweise darauf beruhen, dass die zu detektierende Substanz (Analyt) mit bestimmten Nachweisreagentien reagieren kann, wobei eine Farbänderung des Reaktionsgemisches eintritt. Systeme zum Nachweis derartiger Farbreaktionen und somit zum Nachweis der entsprechenden Analyten sind beispielsweise aus CA 2,050,677 bekannt.

Die beschriebenen Nachweisverfahren beruhen also überwiegend darauf, dass ein Patient zunächst eine entsprechende Probe der zu untersuchenden Körperflüssigkeit entnimmt (wobei es sich beispielsweise um eine Blutprobe oder eine Urinprobe handelt) und diese dann entsprechend mittels der Testvorrichtung untersucht. Dieses Verfahren beinhaltet jedoch verschiedene Nachteile. So ist zum einem dieses Verfahren äußerst aufwändig und setzt mehrere Handhabungsschritte voraus. So muss beispielsweise eine Lanzette bereitgestellt und gespannt werden, anschließend mittels dieser Lanzette eine Hautschicht perforiert werden, dann ein so erzeugter Blutstropfen auf einen Teststreifen aufgetragen werden und anschließend dieser Teststreifen mittels eines entsprechenden Gerätes ausgewertet werden. Für viele Patienten, insbesondere ältere Menschen und Kinder, sind diese Handhabungsschritte oftmals nur schwer durchzuführen, da die Patienten beispielsweise in ihrer motorischen Fähigkeit und ihrem Sehvermögen eingeschränkt sind. Weiterhin lassen sich diese Verfahrensschritte nur in wenigen Fällen diskret durchführen, so dass beispielsweise ein Schutz der Privatsphäre des Patienten bei einer Messung am Arbeitsplatz nur unzureichend gewahrt ist. Auch kann eine Fehlbedienung des Messverfahrens leicht zu falschen Messwerten führen, mit teilweise fatalen Folgen einer auf den Messergebnissen aufbauenden falschen Medikation.

Aus dem Stand der Technik sind daher Systeme bekannt, welche in ein Körpergewebe implantiert werden können und welche beispielsweise kontinuierlich Messwerte liefern. So offenbart beispielsweise US 6,892,085 B2 ein gekapseltes Glucosesensorsystem, welches einen Glucosesensor und eine Schutzkapselung umfasst. Dabei sind drei Elektroden, eine Arbeitselektrode, eine Gegenelektrode und eine Referenzelektrode vorgesehen, welche auf einer Seite eines dünnen Substrats aufgebracht sind. Zur besseren Implantierbarkeit ist diese Elektrodenanordnung in eine Hohlnadel eingebracht, welche in Körpergewebe eingestochen wird.

Auch US 5,591,139 offenbart ein implantierbares Mikronadelsystem, mittels dessen für Diagnosezwecke Substanzen aus lebenden Gewebe entnommen werden können. Dabei wird ein geätztes dreidimensionales Substrat verwendet.

Insgesamt sind jedoch die aus dem Stand der Technik bekannten implantierbaren Sensoren bezüglich ihres Aufbaus und ihrer Herstellung äußerst aufwendig. Geht man davon aus, dass es sich bei diesen Sensoren um nur für kurze Zeit (typischerweise circa eine Woche) verwendbare Einwegsensoren handelt, so wird deutlich, dass die bei den aus dem Stand der Technik bekannten Sensoren eingesetzten Verfahren derartigen Anforderungen an Einwegartikel nicht gerecht werden.

So ist beispielsweise für die Herstellung des aus US 5,591,139 bekannten Sensors ein aufwändiges Mikrostrukturierungsverfahren erforderlich, insbesondere ein lithographisches Verfahren. Derartige Verfahren sind jedoch mit der Herstellung kostengünstiger Einwegartikel nicht vereinbar. Auch zur Herstellung des aus US 6,892,085 B2 bekannten Sensors sind aufwändige Strukturierungsverfahren erforderlich, da die Elektrodenpads sorgfältig strukturiert werden müssen. Angesichts der geringen Größe dieser Elektroden sind dafür ebenfalls lithographische Verfahren erforderlich, was die Kosten zur Herstellung derartiger Sensoren wiederum in die Höhe treibt.

Auch sind lithographische Verfahren, insbesondere das mit diesen Verfahren verbundene Ätzen von Metallschichten, nicht immer so zuverlässig, wie dies für die Herstellung medizintechnischer Produkte erforderlich ist. Insbesondere kann es vorkommen, dass einzelne Elektroden noch durch "Brücken" miteinander verbunden sind, so dass die Funktionalität der Sensoren aufgrund von Herstellungsproblemen leicht beeinträchtig oder sogar ganz verhindert sein kann. Ein weiterer Nachteil der aus dem Stand der Technik bekannten Sensoren, wie beispielsweise der aus der US 6,892,085 B2 und US 5,591,139 bekannten Sensoren, besteht weiterhin in der Verwendung einer Hohlnadel bzw. Kapillare. Die Sensoren sind in diesen Fällen in eine Kapillare eingebracht, welche die zu untersuchende Körperflüssigkeit zum Sensor hintransportiert. Nachteilig daran ist jedoch, dass die Kapillare einen ungehinderten Zugang der Analytlösung zu den Elektroden erschwert. Insbesondere kann es dadurch auch zu lokalen Konzentrationsverfälschungen kommen, welche dazu führen, dass Messergebnisse nicht den tatsächlichen Konzentrationsverhältnissen in der Körperflüssigkeit entsprechen. Dabei spielen auch komplexe Diffusionsvorgänge und Strömungsvorgänge in den Kapillaren eine Rolle und tragen zur Verfälschung bei.

### Aufgabe der Erfindung:

Aufgabe der Erfindung ist es somit, einen Sensor zur Verfügung zu stellen, welcher einfach und kostengünstig mittels eines zuverlässigen Herstellungsverfahrens herstellbar ist und welcher die Nachteile der aus dem Stand der Technik bekannten Sensoren und Verfahren nach Möglichkeit vermeidet.

### Darstellung der Erfindung:

Diese Aufgabe wird durch die Erfindung mit den Merkmalen der unabhängigen Ansprüche gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt der Beschreibung gemacht.

Es wird ein implantierbarer Sensor zur Ermittlung einer Konzentration mindestens eines Analyten in einem Medium, insbesondere in einem Körpergewebe und/oder einer Körperflüssigkeit, vorgeschlagen. Bei dem Analyten kann es sich beispielsweise um Glucose, Triglyceride, Laktat, Hormone oder andere Analyten handeln, welche insbesondere in der Medizin eine Rolle spielen. Alternativ oder zusätzlich kann der Sensor jedoch auch zur Messung anderer Arten von Analyten eingesetzt werden. Insbesondere beruht der Sensor auf der Verwendung eines elektrochemischen Messverfahrens.

Ein Grundgedanke der Erfindung besteht darin, den implantierbaren Sensor derart auszugestalten, dass Elektroden auf einem Substrat in mindestens zwei Elektrodenebenen, beispielsweise auf einer Vorder- und einer Rückseite, aufgebracht werden. Die Messung der Analytkonzentration erfolgt dann vorzugsweise nach Benetzung des Sensors durch amperometrische Messung zwischen den mindestens zwei Elektroden (Arbeits- und Gegenelektrode), insbesondere mittels einer Gleichspannung. Eine Referenzelektrode zur stromlosen Messung des Arbeitselektrodenpotenzials kann zusätzlich verwendet werden.

Um diesen dreidimensionalen Aufbau zu verwirklichen, weist der implantierbare Sensor einen Schichtaufbau auf. Dieser Schichtaufbau verfügt über mindestens ein isolierendes Trägersubstrat und mindestens zwei Elektroden, welche für das elektrochemische Messverfahren eingesetzt werden können. Diese mindestens zwei Elektroden weisen mindestens eine Arbeitselektrode und mindestens eine weitere Elektrode auf, wobei die mindestens eine weitere Elektrode insbesondere mindestens eine Gegenelektrode und/oder und mindestens eine Referenzelektrode umfassen sollte.

Die Arbeitselektrode und die mindestens eine weitere Elektrode sind dabei in unterschiedlichen Schichtebenen des Schichtaufbaus angeordnet. Insbesondere ist unter "unterschiedlichen Schichtebenen" dabei zu verstehen, dass zwischen den mindestens zwei Elektroden mindestens ein isolierendes Trägersubstrat angeordnet ist, so dass mindestens zwei der mindestens zwei Elektroden durch das isolierende Trägersubstrat getrennt sind. Somit wird, im Gegensatz zum oben beschriebenen Stand der Technik, bei diesem Aufbau eines implantierbaren Sensors die _{"}dritte Dimension" mitgenutzt.

Zwar sind aus dem Stand der Technik elektrochemische Zellen, beispielsweise elektrochemische Mikrozellen, bekannt, bei welchen Elektroden auf gegenüberliegenden Seiten eines Trägers angeordnet sind. In diesem Fall handelt es sich jedoch bei dem Träger um ein Elektrolytmaterial, insbesondere einen Feststoffelektrolyten. Im Gegensatz dazu weist das mindestens eine isolierende Trägersubstrat des erfindungsgemäßen elektrochemischen Sensors ein elektrisch nicht leitendes Material auf. Dies soll insbesondere bedeuten, dass, wenn an zwei auf entgegengesetzten Seiten des mindestens einen isolierenden Trägersubstrats angeordnete Elektroden eine Spannung von bis zu ca. 1-2 Volt angelegt wird, Ströme von nicht mehr als einem Milliampere, vorzugsweise von nicht mehr 100 Mikroampère, und besonders bevorzugt von nicht mehr als 10 Mikroampère, fließen. Dadurch ist gewährleistet, das der Messstrom aufgrund des elektrochemischen Messverfahrens erheblich grö-βer ist als der Strom, welcher trotz der isolierenden Eigenschaften des elektrisch nicht leitenden Materials des mindestens einen isolierenden Trägersubstrats durch das mindestens eine isolierende Trägersubstrat fließt.

Der implantierbare Sensor kann auf verschiedene Arten vorteilhaft erfindungsgemäß weitergebildet werden. Die beschriebenen vorteilhaften Weiterbildungen können dabei einzeln oder in Kombination verwendet werden.

So kann der implantierbare Sensor weiterhin mindestens zwei die mindestens zwei Elektroden elektrisch kontaktierende Elektrodenkontaktschichten aufweisen. Dabei kann sich beispielsweise um elektrisch leitende Schichten handeln, z. B. metallische Schichten, insbesondere um Schichten, welche Karbon, Graphit, Gold, Silber, Platin, und/oder Aluminium aufweisen. Auch mehrschichtige Aufbauten sind möglich. Auch organische Leitermaterialien kommen als elektrisch kontaktierende Elektrodenkontaktschichten in Betracht.

Da die mindestens zwei Elektroden in mindestens zwei Schichtebenen des Schichtaufbaus des Sensors angeordnet sind, ist es nunmehr möglich, dass sich die mindestens zwei Elektroden und/oder die mindestens zwei Elektrodenkontaktschichten im Wesentlichen über die gesamte Breite des mindestens einen isolierenden Trägersubstrats erstrecken. So kann das isolierende Trägersubstrat insbesondere eine Längserstreckung sowie eine Breite und eine Länge aufweisen, wobei sich die Elektrodenkontaktschichten und/oder die Elektroden im Wesentlichen in einem Rand des isolierenden Trägersubstrats zum anderen erstrecken. Unter "im Wesentlichen" kann dabei eine Abdeckung des isolierenden Trägersubstrats durch die Elektrodenkontaktschicht von mindestens 80%, vorzugsweise 95% und besonders bevorzugt von 100% verstanden werden. Eine Strukturierung der Elektroden und/oder Elektrodenkontaktschichten ist also aufgrund der Trennung durch den Aufbau in verschiedenen Ebenen nicht mehr erforderlich, so dass auf aufwändig lithographische Strukturierungsverfahren oder Laserstrukturierungsverfahren (z. B. Laserablation) verzichten werden kann. Die Vorteile dieser Möglichkeit werden unten deutlich, wenn ein mögliches Herstellungsverfahren des implantierbaren Sensors im Detail beschrieben wird.

Weiterhin kann der implantierbare Sensor mindestens zwei Isolatorschichten aufweisen, welche die mindestens zwei Elektrodenkontaktschichten zumindest teilweise, vorzugsweise jedoch vollständig, gegen das Medium, insbesondere das Körpergewebe und/oder die Körperflüssigkeit, abdecken und vorzugsweise elektrisch isolieren. Dabei können die beispielsweise mindestens zwei Elektrodenkontaktschichten teilweise von den mindestens zwei Elektroden bedeckt sein, wobei die unbedeckten Bereiche (z. B. nachträglich) durch die mindestens zwei Isolatorschichten bedeckt werden. Vorzugweise werden als Isolatorschichten selbstklebende Folienschichten eingesetzt.

Neben dem oben beschriebenen Schichtaufbau können auch noch weitere, nicht genannte Schichten vorgesehen sein. So kann es sich bei dem mindestens einen isolierenden Trägersubstrat selbst bereits um einen mehrschichtigen Aufbau handeln, beispielsweise um ein isolierendes Trägersubstrat aus einem Substratmaterial, welcher mit weiteren Schichten besichtet ist. So können beispielsweise Substratmaterialen mit zusätzlichen Barriereschichten eingesetzt werden. Besonders bevorzugt ist es, wenn das mindestens eine isolierende Trägersubstrat als Werkstoff ein Polymer, ein isolierendes Polymer, vorzugsweise einen Polyester, aufweist. Zwischen den mindestens einen isolierenden Trägersubstrat und die mindestens zwei Elektrodenkontaktschichten und/oder die mindestens zwei Elektroden können auch weitere Schichten eingebracht sein, beispielsweise leitende oder elektrisch isolierende Schichten.

Der implantierbare Sensor gemäß der obigen Beschreibung kann beispielsweise in eine Kanüle, wie aus dem Stand der Technik bekannt, eingesetzt werden. Besonders bevorzugt ist es jedoch, wenn der implantierbare Sensor unmittelbar, das heißt ohne umgebende Kanüle oder Kapillare in das Medium insbesondere das Körpergewerbe, eingebracht werden kann. Auf diese Weise ist gewährleistet, dass die in den mindestens zwei Ebenen angeordneten Elektroden frei von Körperflüssigkeit umspült werden können. Zu diesem Zweck kann der implantierbare Sensor mindestens eine den Schichtaufbau ganz oder teilweise umschließende Membranschicht aufweisen. Vorteilhafterweise weist diese mindestens eine Membranschicht zumindest teilweise eine Durchlässigkeit für den mindestens einen Analyten, welcher nachgewiesen werden soll, auf. Beispielsweise kann die mindestens eine Membranschicht eine Durchlässigkeit für Glucose, Laktat, Triglyceride und/oder weitere Analyten aufweisen. Dabei sollte jedoch vorteilhafterweise die mindestens eine Membranschicht undurchlässig sein für bei dem elektrochemischen Messverfahren eingesetzte Hilfschemikalien, beispielsweise für eingesetzte Enzyme, welche auf eine oder mehrere der Elektroden aufgebracht sind. Beispielsweise kann es sich dabei um Glucoseoxidase handeln. Somit gewährleistet die Membranschicht auch, dass diese Hilfschemikalien, welche teilweise von erheblicher Toxizität sind (z. B. stellt Glucoseoxidase ein Zellgift da), nicht in das Körpergewebe gelangen und da Schaden anrichten können.

Die mindestens eine Membranschicht kann beispielsweise den Bereich, in welchem die Elektroden aufgebracht sind, umschließen. Beispielsweise kann die mindestens eine Membranschicht ein Polyurethan aufweisen. Auch ein mehrschichtiger Membranschichtaufbau ist möglich. Beispielweise können für das Aufbringen des Polyurethans nasschemische Verfahren, z. B. Tauchverfahren oder Sprühverfahren, oder auch andere bekannte Beschichtungsverfahren eingesetzt werden.

Die Ausgestaltung der mindestens zwei Elektroden kann auf verschiedene Weisen erfolgen. Insbesondere können die mindestens zwei Elektroden, wie oben beschrieben, mindestens eine Arbeitselektrode und mindestens eine weitere Elektrode, welche mindestens eine Gegenelektrode und mindestens eine Referenzelektrode aufweist, umfassen. Insbesondere sollte die mindestens eine Gegenelektrode ein zu einem Redoxverhalten der mindestens einen Arbeitselektrode umgekehrtes Redoxverhalten aufweisen. Eine Gegenelektrode und eine Referenzelektrode können auch als gemeinsame Elektrode ausgebildet sein, vorzugsweise als eine gemeinsame Elektrode, deren Fläche größer ist als die Fläche der mindestens einen Arbeitselektrode. Aus dem Stand der Technik sind Beispiele für die Verwendung von Elektrodenmaterialien für elektrochemische Messverfahren bekannt. So können beispielsweise Elektroden mit Enzymen oder anderen chemischen Hilfsstoffen beschichtet werden, welche spezifisch für den nachzuweisenden Analyten sind. Beispielsweise kann zum Nachweis von Glucose Glucoseoxidase (GOD) eingesetzt werden, welche Glucose in Gluconolakton umwandelt. Die dabei freiwerdenden Ladungsträger werden nachgewiesen. Um diesen Nachweis zu ermöglichen, werden die überspannungsreduzierenden Materialien eingesetzt, welche quasi als "Ladungsvemittler" zwischen dem Medium und den Elektroden dienen.

Vieler dieser überspannungsreduzierenden Materialien sind jedoch gesundheitsschädlich. Insbesondere haben sich dabei Mediatoren als toxisch erwiesen, so dass eine Inmobilisierung dieser überspannungsreduzierenden Materialien für einen Einsatz in implantierbaren Sensoren in vielen Fällen erforderlich ist. Zur Immobilisierung kann beispielsweise eine kovalente Bindung an die Elektrode und/oder eine Schicht der Elektrode, beispielsweise eine Metallschicht, erfolgen. Insbesondere kann diese Technik zur Immobilisierung von Mediatoren eingesetzt werden. Eine zweite Möglichkeit besteht darin, das Überspannungsreduzierende Material in eine unlösliche Schicht zu integrieren, welche unlöslich ist in der den implantierbaren Sensor im implantierten Zustand umgebende Flüssigkeit, insbesondere der Körperflüssigkeit. Dies kann beispielsweise bei der Verwendung von Braunstein erfolgen, welcher als Paste auf die Elektrode aufgetragen wird und dann nach dem Trocknen unlöslich ist.

Als Mediatoren können beispielsweise Nitroseaniline, Hexacyanoferrat, Ferrocene oder andere bekannte Mediatoren eingesetzt werden. Neben Braunstein lassen sich auch andere Materialien einsetzen.

Neben der beschriebenen Ausgestaltung der mindestens einen Arbeitselektrode kann auch die Ausgestaltung der mindestens einen Referenzelektrode und/oder der Ausgestaltung der mindestens einen Gegenelektrode auf verschiedene Weisen erfolgen. So sollte die mindestens eine Referenzelektrode ein Elektronensystem mit einem elektrochemischen Potenzial aufweisen, welches sich in einem Arbeitsbereich des implantierbaren Sensor nicht oder nur unwesentlich ändert. So sollte sich beispielsweise bei einer typischen Spannungsbelastung (also einer Spannung zwischen Arbeitselektrode und Referenzelektrode) von typischerweise 300-500 Millivolt, z.B. 450 Millivolt, das elektrochemische Potenzial der mindestens einen Referenzelektrode vorzugsweise um nicht mehr als 1 Millivolt, vorzugsweise um eine nicht als im 1 Mikrovolt-Bereich liegende Änderung, ändern. Auf diese Weise ist sichergestellt, dass die Referenzelektrode als wirkliche Referenz wirkt, mit deren Potenzial das elektrochemische Potentziel der mindestens einen Arbeitselektrode verglichen werden kann.

Grundsätzlich lassen sich für die Referenzelektrode eine Vielzahl von Materialien und/oder Materialkombinationen einsetzten. Also besonders vorteilhaft hat sich dabei ein Silber/Silberchlorid(Ag/AgCl)-Elektrodensystem erwiesen. Auch andere Elektrodensysteme sind prinzipiell einsetzbar, sind jedoch weniger üblich, wie z. B. HgCl₂- Elektrodensysteme.

Auch die mindestens eine Gegenelektrode kann auf eine Vielzahl von verschiedenen Weisen ausgestaltet sein. Dabei sollte jedoch gewährleistet sein, dass die mindestens eine Gegenelektrode ein zu einem Redoxverhalten der mindestens einen Arbeitselektrode umgekehrtes Redoxverhalten gegenüber der umgebenden Flüssigkeit aufweist. Wenn also an der Arbeitselektrode eine Oxidation stattfindet, sollte an der Gegenelektrode eine Reduktion stattfinden und umgekehrt. Prinzipiell lassen sich reine Metalle als Gegenelektroden einsetzen, wie beispielsweise Platin. Dies hat jedoch den Nachteil, dass an derartigen Metallelektroden typischerweise eine Gasbildung auftritt, beispielsweise eine Bildung von Wasserstoff oder Sauerstoff. Eine derartige Gasbildung ist jedoch bei implantiertem Sensor im Körpergewebe unerwünscht. Insofern ist es auch hier wiederum von Vorteil, wenn ein Elektrodensystem, insbesondere ein Redoxelektrodensystem, eingesetzt wird, bei welchem eine Gasbildung vermieden wird. Insbesondere lässt sich auch hier wieder eine Ag/AgCl-Elektrodensystem einsetzen. Dabei wird beispielsweise AgCl reduziert. Daraus ist ersichtlich, dass die Gegenelektrode im Betrieb des Sensors verbraucht wird. Ist die Gegenelektrode aufgebraucht, findet wiederum häufig eine Gasbildung statt, so dass der implantierbare Sensor im Betrieb in der Regel eine begrenzte Lebensdauer aufweist. Dementsprechend ist auch von Vorteil, wenn die mindestens eine Gegenelektrode von ihrer Fläche her erheblich größer ausgestaltet wird als die mindestens eine Arbeitselektrode.

Die Art und Weise der Aufbringung der Elektroden auf die Elektrodenkontaktschichten kann auf unterschiedliche Weise erfolgen, abhängig vom verwendeten Elektrodenmaterial. Werden beispielsweise reine Metalle als Elektrodenmaterialien eingesetzt, so lassen sich beispielsweise Folienverfahren (z. B. Laminieren) einsetzen oder nasschemische Verfahren, physikalische Aufbringungsverfahren (physical vapor deposition, PVD, z. B. Aufdampfen oder Aufsputtern) oder auch chemische Aufbringungsverfahren (chemical vapor deposition, CVD). Braunstein (MnO₂/C) lässt sich beispielsweise als Beschichtung aufbringen, beispielsweise als Pastenbeschichtung. Hierbei können unterschiedliche, dem Fachmann bekannte Beschichtungsverfahren eingesetzt werden, beispielsweise Siebdruck, Rakeln, Düsenbeschichtung oder ähnliches. Dabei kann beispielsweise ein Enzym bereits mit der Paste vermischt werden, so dass Enzym und Braunstein in einem Schritt aufgebracht werden können. Alternativ kann auch zunächst Braunstein aufgebracht werden, woraufhin in einem nachfolgenden Schritt das Enzym, beispielsweise Glucoseoxidase (GOD), z. B. aufdispensiert wird oder in einem anderen nasschemischen Schritt aufgebracht wird. Entsprechend werden auch die anderen Elektroden aufgebracht. Typische E-lektrodenschichtdicken liegen im Bereich von 10 Mikrometern, können jedoch bis in den Bereich von hundert bis einigen hundert Mikrometern liegen. Auch dünnere Elektrodenschichten sind denkbar.

Erfindungsgemäß kann der implantierbare Sensor und/oder eine Vorrichtung, welche den implantierbaren Sensor enthält, für eine kontinuierliche Ermittlung einer Konzentration mindestens eines Analyten im Körpergewebe und/oder einer Körperflüssigkeit eingesetzt werden. Unter "kontinuierlich" kann dabei beispielsweise verstanden werden, dass über einen bestimmten Messzeitraum hinweg, beispielsweise eine Woche, in regelmäßigen Abständen (z. B. alle fünf Minuten oder jede Stunde) oder auch permanent, d. h. mit einer zeitlichen Auflösung, welche lediglich durch die zeitliche Auflösung eines Messgeräts begrenzt ist, Analytkonzentrationen bestimmten werden.

Eine Problematik besteht jedoch bei einer kontinuierlichen Messung in einer möglichen Drift der Vorrichtung und/oder des Sensors über den Messzeitraum. Üblicherweise erfolgt eine kontinuierliche Messung dadurch, dass zunächst mittels eines "konventionellen" Messverfahrens (z. B. Entnahme eines Blutstropfens und Messung des Blutstropfens) eine Referenzmessung durchgeführt wird, welche dann mit dem vom implantierten Sensor gelieferten Messwert abgeglichen wird. Anschließend folgt über den Messzeitraum hinweg eine Messung unter Zugrundelegung des anfänglichen Referenzmesswertes. Ändert sich dabei jedoch die Eigenschaft des implantierbaren Sensors, beispielsweise aufgrund einer Drift eines elektrochemischen Potenzials, insbesondere des elektrochemischen Potenzials einer der mindestens einen Arbeitelektrode und/oder der mindestens einen Referenzelektrode, so ist diese Messung fehlerbehaftet und unterliegt einer Drift.

In der Praxis hat es sich dabei gezeigt, dass insbesondere die mindestens eine Membranschicht und das für die mindestens eine Arbeitselektrode verwendete Elektrodenmaterial bezüglich der Drift kritische Punkte darstellen. Die Verwendung einer Braunsteinpaste, insbesondere einer mit einem Enzym (z. B. Glucoseoxidase) vermengten Braunsteinpaste, welche aufgetragen und anschließend getrocknet wird, hat sich hierbei als vorteilhaft erwiesen, da diese Auswahl eine Drift minimiert. Auch die Verwendung der beschriebenen vorteilhaften Polyurethan-Membran minimiert die Drift zusätzlich.

Der erfindungsgemäße implantierbare Sensor kann weiterhin dahingehend weitergebildet werden, dass dieser eine Einführspitze zum Einstechen des implantierbaren Sensors in das Medium, insbesondere in ein Fettgewebe (z. B. in ein interstitielles Fettgewebe), aufweist. Dabei kann beispielsweise die oberste Hautschicht durchdrungen werden und der Sensor zumindest teilweise unter die Lederhaut geschoben werden.

Diese Einführspitze kann auf verschiedene Weise ausgestaltet sein. Wie oben erwähnt und auch aus dem Stand der Technik bekannt, können beispielsweise Kanülen eingesetzt werden. Insbesondere ist es jedoch erfindungsgemäß bevorzugt, wenn der Sensor selbst, also beispielsweise der Schichtaufbau selbst, eine derartige Einführspitze aufweist.

Die Problematik bisheriger Sensoren besteht jedoch darin, dass diese üblicherweise sehr dünn und breit ausgelegt sind. Dadurch verbiegt sich jedoch der Schichtaufbau beim Einstechen, so dass die für das Einstechen des Sensors erforderliche Kraft nicht über den Sensor übertragen werden kann, dass dieser zuvor abknickt. Der erfindungsgemäße Sensor, bei welchem die Elektrodenkontaktschichten vorzugsweise großflächig aufgebracht werden und nicht strukturiert werden müssen, ermöglicht jedoch einen Aufbau mit einem hohen Aspektverhältnis. Unter einem Aspektverhältnis soll dabei das Verhältnis zwischen der Höhe und der Breite des isolierenden Trägersubstrats und/oder des gesamten Schichtsaufbaus verstanden werden. So können beispielsweise isolierende Trägersubstrate und/oder Schichtaufbauten verwendet werden, bei welchen dieses Aspektverhältnis, welches im Folgenden als k bezeichnet wird, mindestens 0,3, vorzugsweise 0,5 und besonders bevorzugt mindestens 0,9 beträgt.

Wollte man derartige Aspektverhältnisse mit herkömmlichen Sensoren, bei welchen die Elektrodenkontaktschichten strukturiert sind, erreichen, müssten, da strukturierte Elektroden eine hohe Breite des isolierenden Trägersubstrats voraussetzen, auch sehr dicke Sensoren verwendet werden. Dies bedeutet wiederum einem hohen Querschnitt des Einstichkanals des Sensors. Durch den erfindungsgemäßen Aufbau, bei welchem ein hohes Aspektverhältnis unter gleichzeitiger Minimierung der Einstichfläche erreicht wird, wird dieser Nachteil vermieden.

Besonders bevorzugt ist es, wenn der Schichtaufbau des erfindungsgemäßen Sensors ein gestufter Schichtaufbau ist. Dabei sollten mindestens zwei isolierende Trägersubstrate vorhanden sein, wobei mindestens zwei dieser isolierenden Trägersubstrate eine Stufe bilden. Insbesondere ist es bevorzugt, wenn diese Stufe in Längserstreckungsrichtung des implantierbaren Sensors (also in Einstichrichtung) ausgebildet ist. Zu diesem Zweck kann beispielsweise einer der mindestens zwei isolierenden Trägersubstrate kürzer ausgebildet sein als ein zweiter der isolierenden Trägersubstrate, wodurch vorzugsweise an der Spitze des Sensors oder in der Nähe der Spitze eine Stufe entsteht. Dadurch ist es möglich, beispielsweise drei Elektroden auszubilden, welche in drei verschiedenen Schichtebenen vorgesehen sind. Beispielsweise kann dabei mindestens eine dieser Elektroden in der Ebene der Stufe, insbesondere an der Stufe selbst, vorgesehen sein. Auf diese Weise wird die bereits oben beschriebene Sandwichstruktur weiter in die dritte Dimension erweitert.

In der Praxis haben sich aufgrund der besonderen Eigenschaften und der besonders einfachen Herstellung insbesondere zwei Schichtaufbauten bewährt, welche jedoch gegebenenfalls um zusätzliche, nicht erwähnte Schichten erweitert werden können:
Bei einem ersten Schichtaufbau wird zunächst ein isolierendes Trägersubstrat zwischen eine erste Elektrodenkontaktschicht und eine zweite Elektrodenkontaktschicht eingebettet. Dieses "Einbetten" kann auf verschiedene Weisen erfolgen, wobei beispielsweise Laminierverfahren oder andere Verfahren (siehe unten) eingesetzt werden. Auch ist durch den Begriff "Einbetten" nichts über die zeitliche Reihenfolge des Schichtsaufbaus ausgesagt, so dass z. B. zunächst eine erste Elektrodenkontaktschicht, dann das isolierende Trägersubstrat und anschließend die zweite Elektrodenkontaktschicht aufgebracht werden können. Auch andere Reihenfolgen sind möglich.

Weiterhin wird auf der dem isolierenden Trägersubstrat abgewandten Seite der ersten Elektrodenkontaktschicht mindestens eine erste Elektrode aufgebracht und auf der den isolierenden Trägersubstraten abgewandten Seite der zweiten Elektrodenkontaktschicht mindestens eine zweite Elektrode. Beispielsweise kann auf einer Seite die mindestens eine Arbeitselektrode aufgebracht werden und auf der gegenüberliegenden Seite auf der zweiten Elektrodenkontaktschicht die mindestens eine Referenzelektrode und/oder die mindestens eine Gegenelektrode, beispielsweise als gemeinsame Elektrode. Auf diese Weise entsteht ein "Sandwichaufbau".

Bei einem zweiten bevorzugten Schichtaufbau wird das oben beschriebene "Stufenkonzept" verwirklicht. Dazu wird ein erstes isolierendes Trägersubstrat zwischen einer ersten Elektrodenkontaktschicht und einer zweiten Elektrodenkontaktschicht eingebettet. Auf die dem isolierenden Trägersubstrat abgewandte Seite der zweiten Elektrodenkontaktschicht wird ein zweites isolierendes Trägersubstrat aufgebracht. Wiederum sei darauf hingewiesen, dass hier lediglich nur der Schichtaufbau beschrieben wird, wobei die zeitliche Reihenfolge des Aufbringens der einzelnen Schichten variieren kann.

Das zweite isolierende Trägersubstrat ist in einer Längserstreckungsrichtung des implantierbaren Sensors (Einstichrichtung) kürzer als das erste isolierende Trägersubstrat, so dass sich eine Stufe ausbildet. Auf der dem isolierenden Trägersubstrat abgewandten Seite des zweiten isolierenden Trägersubstrats ist eine dritte Elektrodenkontaktschicht aufgebracht. Nunmehr sind Elektroden in drei Schichtebenen vorgesehen: Eine erste Elektrode auf der ersten Elektrodenkontaktschicht, eine zweite Elektrode auf der zweiten Elektrodenkontaktschicht, insbesondere im Bereich der Stufe, und eine dritte Elektrode auf der dritten Elektrodenkontaktschicht. Diese Elektroden sind dabei jeweils auf der dem zugehörigen isolierenden Trägersubstrat abgewandten Seite der Elektrodenkontaktschichten angeordnet.

Weiterhin wird eine Vorrichtung zur Ermittlung einer Konzentration von mindestens einem Analyten in einem Medium, insbesondere einem Körpergewebe und/oder eine Körperflüssigkeit, vorgeschlagen. Die erfindungsgemäße Vorrichtung umfasst mindestens einen implantierbaren Sensor gemäß der obigen Beschreibung der möglichen Ausgestaltungen. Weiterhin umfasst die mindestens eine Vorrichtung mindestens eine Spannungsmessvorrichtung zur Messung einer Spannung zwischen der mindestens einen Arbeitselektrode und der mindestens einen Referenzelektrode. Weiterhin kann mindestens eine Strommessvorrichtung zur Messung eines Stroms zwischen der mindestens einen Gegenelektrode und der mindestens einen Arbeitselektrode vorgesehen sein. Zusätzlich kann die Vorrichtung weiterhin eine Regelvorrichtung umfassen, welche ausgestaltet ist, den Strom zwischen der mindesten eine Gegenelektrode und der mindestens einen Arbeitselektrode zu regeln, dergestalt, dass die zwischen der mindestens einen Arbeitselektrode und der mindestens einen Referenzelektrode gemessene Spannung gerade gleich einer vorgegebenen Sollspannung ist. Dem Fachmann sind weitere Verfahren und Vorrichtungen zur Bestimmung einer elektrochemischen Potenzialdifferenz zwischen der mindestens einen Arbeitselektrode und der mindestens einen Gegenelektrode sowie eine konkrete elektronische Ausgestaltung derartiger Schaltungen bekannt.

Der beschriebene erfindungsgemäße Sensor kann beispielsweise für eine kontinuierliche Ermittlung einer Konzentration mindestens eines Analyten im Körpergewebe und/oder einer Körperflüssigkeit eingesetzt werden. Zu diesem Zweck kann beispielsweise der erfindungsgemäße Sensor, z. B. als Bestandteil der erfindungsgemäßen Vorrichtung in einer der beschriebenen Ausgestaltungen, durch Einstechen in das Körpergewebe implantiert werden. Anschließend kann dem Sensor ein gewisse Zeit zur Verfügung gestellt werden, innerhalb derer sich im Bereich des Sensors und des umgebenen Körpergewebes ein (zumindest annäherndes) Gleichgewicht einstellt. Beispielsweise kann während dieser Zeit, welche z. B. eine Stunde betragen kann, eine Quellung einzelner oder aller Schichten des Sensors erfolgen. Anschließend kann der Patient eine Kalibrationsmessung durchführen, bei welcher, wie oben beschrieben, mittels eines konventionellen Verfahrens eine Analytkonzentration in der Körperflüssigkeit bestimmt wird, beispielsweise eine Glucosekonzentration in einem Blutstropfen. Die dabei ermittelten Daten werden der erfindungsgemäßen Vorrichtung übermittelt, beispielsweise durch manuelles Eingeben oder auch durch elektronische Datenübertragung (z. B. mittels eines Kabels oder einer drahtlosen Verbindung). Dadurch wird der Vorrichtung ein Kalibrationspunkt zur Verfügung gestellt, und die erfindungsgemäße Vorrichtung kann die eingegebenen Messwerte gegen Messwerte, welche der implantierte Sensor liefert, abgleichen. Anschließend können der implantierte Sensor und die erfindungsgemäße Vorrichtung beispielsweise über einen Zeitraum von einer Woche verwendet werden, wobei beispielsweise alle 5 Minuten oder auch ununterbrochen (siehe oben) eine Messung erfolgt. Die von der erfindungsgemäßen Vorrichtung ermittelten Messwerte können beispielsweise an den Patienten ausgegeben werden, oder sie können auch anderen Systemen zur Verfügung gestellt werden, beispielsweise Medikationssystemen. So kann beispielsweise die erfindungsgemäße Vorrichtung unmittelbar mit einer Insulinpumpe verbunden werden, welche eine Insulindosierung an die gemessenen Blutglucosekonzentrationen anpasst. Nach Ablauf der Messzeit kann die komplette Vorrichtung ausgetauscht werden, oder es kann auch lediglich der erfindungsgemäße Sensor gegen einen neuen, unverbrauchten Sensor ausgetauscht werden.

Weiterhin wird ein Verfahren zur Herstellung eines implantierbaren Sensors, insbesondere eines implantierbaren Sensors gemäß der obigen Beschreibung, welcher zur Ermittlung einer Analytkonzentration in einem Medium, insbesondere in Körpergewebe und/oder einer Körperflüssigkeit, geeignet ist, vorgeschlagen. Das Verfahren weist folgende Schritte auf, wobei die Schritte nicht notwendigerweise in der nachfolgenden wiedergegebenen Reihenfolge durchgeführt werden müssen. Auch können verschiedene Verfahrensschritte wiederholt oder parallel durchgeführt werden, und es können zusätzliche, nicht aufgeführte Verfahrensschritte durchgeführt werden.

In einem Verfahrensschritt wird ein Schichtaufbau erzeugt, insbesondere ein "Sandwich"-"Schichtaufbau" oder ein ähnlicher mehrschichtiger Aufbau gemäß der obigen Beschreibung, wobei zwei Elektrodenkontaktschichten, insbesondere zwei Metallschichten, großflächig in mindestens zwei verschiedenen Schichtebenen auf mindestens eine mindestens ein nicht elektrisch leitendes Material umfassende Trägerfolie aufgebracht werden. Für die Metallschichten und die mindestens eine Trägerfolie lassen sich beispielsweise die oben beschriebenen Materialien einsetzten. Weiterhin werden mindestens zwei Elektroden auf die mindestens zwei Elektrodenkontaktschichten aufgebracht, wobei wiederum die oben beschriebenen Werkstoffe eingesetzt werden können. Anschließend wird mittels eines Präzisions-Schneideverfahrens der Schichtaufbau in Sensorstreifen geschnitten.

Im Gegensatz zum Stand der Technik erfolgt bei dem erfindungsgemäßen Verfahren also ein Aufbringen von Elektrodenkontaktschichten großflächig und in mindestens zwei verschiedenen Schichtebenen. Eine zusätzliche Strukturierung der mindestens zwei Elektrodenkontaktschichten erfolgt vorzugsweise nicht. Dabei lassen sich aufwändige lithographische Strukturierungsverfahren vermeiden. Dennoch sind die Elektrodenkontaktschichten und somit auch die Elektroden elektrisch voneinander getrennt, da diese in unterschiedlichen Schichtebenen angeordnet sind.

Weiterhin lassen sich mittlerweile in der Technik perfektionierte Präzisionsschneideverfahren mit Schnittbreiten (d. h. minimale Breite der durch das Präzisions-Schneideverfahren erzeugten Streifen) von vorzugsweise unter 1 mm einsetzen. Im Gegensatz zum Stand der Technik müssen jedoch diese Schnittverfahren nun nicht an den bereits strukturierten Elektrodenschichten ausgerichtet werden, was bei aus dem Stand der Technik bekannten Verfahren, bei welchen in der Regel eine lithographische Strukturierung der Elektroden erfolgt, erforderlich war. So musste bei herkömmlichen Verfahren zunächst eine Strukturierung der Elektroden erfolgen, gefolgt von einer Präzisionsausrichtung eines Schneidwerkzeug an den bereits strukturierten Elektroden, gefolgt von einem Schneideverfahren. Bei dem hier beanspruchten Verfahren kann jedoch diese anfängliche Ausrichtung unterbleiben, da der erste Schnitt aufgrund der großflächig strukturierten Elektrodenkontaktschichten nicht oder nur unwesentlich positioniert werden muss.

Bei dem erfindungsgemäßen Verfahren lassen sich verschiedene Schichttechnologien einsetzen. So lassen sich beispielsweise Laminierverfahren einsetzen, insbesondere zum schichtweisen Aufbringen der Trägerfolien, von Metallschichten und/oder Isolatorschichten (z. B. Klebefolien). Verschiedene Laminierverfahren sind dem Fachmann bekannt. Dabei lassen sich auch Rolle-zu-Rolle-Folienverfahren (englisch reel-to-reel) einsetzen. Weiterhin lassen sich, insbesondere für die Aufbringung von organischen Dünnschichten oder metallischen Dünnschichten, auch physikalische Verfahren (z. B. physical vapor deposition, PVD) und/oder chemische Verfahren (chemical vapor deposition, CVD) einsetzen und/oder nasschemische Beschichtungsverfahren. Insbesondere die beschriebenen Rolle-zu-Rolle-Folienverfahren gewährleisten, dass das erfindungsgemäße Verfahren, insbesondere zur Herstellung des erfindungsgemäßen implantierbaren Sensors, im Vergleich zu im Stand der Technik bekannten Verfahren äußerst kostengünstig und zuverlässig ist. Auf diese Weise lassen sich Trägerfolien, metallische Folien, organische Schichten und/oder Isolatorschichten, aufbringen. Insbesondere lassen sich auch die beiden oben beschriebenen, besonders bevorzugten Schichtaufbauten mit einer Trägerfolie oder zwei Trägerfolien (Stufenaufbau) auf diese Weise mittels des erfindungsgemäßen Verfahrens realisieren.

Der erfindungsgemäße implantierbare Sensor, die Vorrichtung, die Verwendung und das erfindungsgemäße Herstellungsverfahren in einer der beschriebenen Ausgestaltungen bieten gegenüber aus dem Stand der Technik bekannten Vorrichtungen und Verfahren eine Reihe von Vorteilen, welche teilweise bereits beschrieben worden sind. Insbesondere lässt sich eine geometrische Anordnung auf zwei gegenüberliegenden Seiten auf (Vorder- und Rückseite) eines isolierenden Trägersubstrats, z. B. eines isolierenden Trägersubstrats aus Kunststoff, realisieren. Bedingt durch neue Schneideverfahren mit reduzierter Schnittbreite ist es möglich, die Elektrodenabstände in vergleichbarer Größenordnung wie bei einer Anordnung einer ebenen Fläche aufzubringen. Das elektrochemische Verhalten der erfindungsgemäßen Sensoren wird demnach durch die Anordnung auf der Vorder- und Rückseite eines Substrats nicht nachteilig beeinflusst.

Weiterhin ist das beschriebene Herstellungsverfahren äußerst kostengünstig und kann auf aufwändige lithographische Strukturierungsverfahren oder Laserablation verzichten. Die Elektrodenflächen werden allein durch Schneid- und Laminierprozesse definiert, und es lassen sich kontinuierliche, kostengünstige Fertigungsverfahren einsetzen.

Auf ein aufwändiges Positionierverfahren zur Positionierung der Einzelelektroden kann verzichtet werden. Dies ist insbesondere bei miniaturisierten Sensoren (mit daraus resultierendem kleinen, das heißt schmerzfreierem Einstichkanal) mit Breiten von unter einem Millimeter von Vorteil. Weiterhin sind die beschriebenen Sensoren sowohl in physiologischen Lösungen mit hohem Elektrolytgehalt als auch in Lösungen mit niedrigem Elektrolytgehalt einsetzbar.

Weitere Einzelheiten und Merkmale der Erfindung ergeben sich aus den nachfolgenden Beschreibungen der Ausführungsbeispiele in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Im Einzelnen zeigt:
- Figur 1: eine perspektivische schematische Darstellung eines ersten Ausführungsbeispiels eines erfindungsgemäßen implantierbaren Sensors;
- Figur 2: ein zweites Ausführungsbeispiel eines erfindungsgemäßen Sensors;
- Figur 3A: eine schematische Darstellung einer erfindungsgemäßen Vorrichtung zur Ermittlung einer Konzentration mindestens eines Analyten unter Verwendung eines Sensors gemäß Figur 1;
- Figur 3B: eine schematische Darstellung eines zweiten Ausführungsbeispiel einer Vorrichtung zur Ermittlung eines Analyten unter Verwendung eines Sensors gemäß Figur 2;
- Figur 4: einen schematischen Ablaufplan eines Herstellungsverfahrens zur Herstellung eines Sensors gemäß Figur 1; und
- Figur 5: einen schematischen Ablaufplan eines Herstellungsverfahrens zur Herstellung eines Sensors gemäß Figur 2.

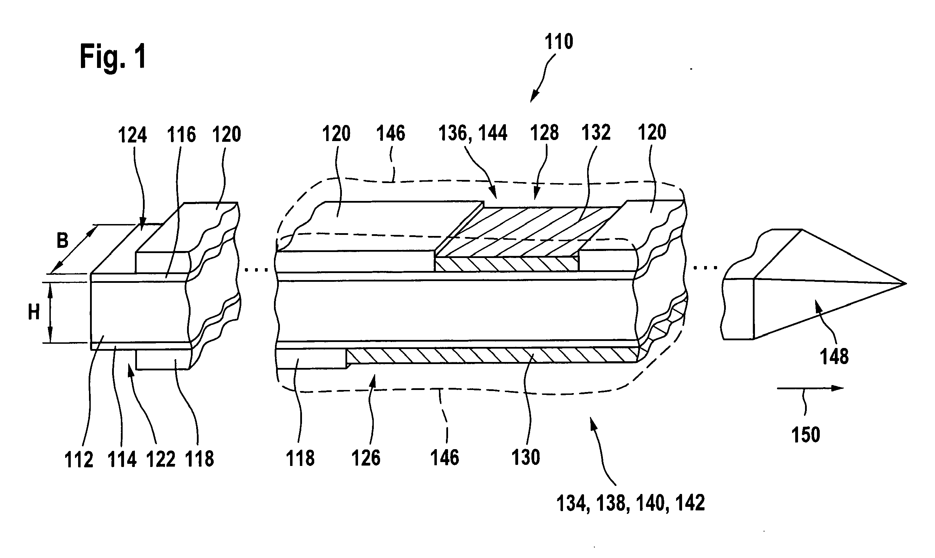

In Figur 1 ist ein mögliches erstes Ausführungsbeispiel eines erfindungsgemäßen implantierbaren Sensors 110 zur Ermittlung einer Glucosekonzentration in einer Körperflüssigkeit dargestellt. Der implantierbare Sensor 110 weist ein isolierendes Trägersubstrat 112 auf, welches in diesem Ausführungsbeispiel aus einem nicht leitenden Polyester besteht. Das isolierende Trägersubstrat 112 hat eine Höhe H von typischerweise 0,8 bis 1 Millimeter, wobei jedoch auch andere Höhen möglich sind, vorzugsweise Höhen im Bereich zwischen 0,2 und 2 Millimetern. Weiterhin weist das isolierende Trägersubstrat 112 eine Breite B auf, welche im Bereich von einem Millimeter liegt. Somit ergibt sich in diesem Ausführungsbeispiel ein Aspektverhältnis H/B von ca. 0,8. Es sind jedoch auch andere Breiten B möglich, insbesondere Breiten zwischen 0,3 Millimetern und 2 Millimetern. Bevorzugt ist es jedoch, ein Aspektverhältnis zu wählen, welches im Bereich von 1,0 liegt, also vorzugsweise von mindestens 0,3, wobei auch das umgekehrte Aspektverhältnis (also 1/k) mindestens 0,3 betragen sollte. Diese Weite des Aspektverhältnisses gewährleistet eine hohe Stabilität des implantierbaren Sensors 110.

Das isolierende Trägersubstrat 112 ist mit einer ersten Elektrodenkontaktschicht 114 und einer auf der gegenüberliegenden Seite angeordneten zweiten Elektrodenkontaktschicht 116 beschichtet. Dabei kann es sich zum Beispiel um eine Folie oder eine andere Schicht eines Metalls wie beispielsweise Gold, Silber, Platin und/oder Aluminium handeln, welche beispielsweise auf das isolierende Trägersubstrat 112 auflaminiert ist. Die Elektrodenkontaktschichten 114, 116 sind großflächig auf das isolierende Trägersubstrat 112 aufgebracht, so dass sich diese über die volle Breite B des isolierenden Trägersubstrats 112 erstrecken.

Auf die Elektrodenkontaktschichten 114, 116 ist jeweils eine Isolatorschicht 118, 120 in Form einer in diesem Ausführungsbeispiel selbstklebenden Folienschicht aufgebracht. Diese Isolatorenschichten 118, 120 enden am linken Ende des implantierbaren Sensors 110 in Figur 1 vor dem Ende des isolierenden Trägersubstrats 112, dergestalt, das in diesem Bereich die erste Elektrodenkontaktschicht 114 und die zweite Elektrodenkontaktschicht 116 freiliegen und elektrische Kontakte 122, 124 bilden. Über diese elektrischen Kontakte 122, 124 können die Elektrodenkontaktschichten 114, 116 elektrisch kontaktiert werden, beispielsweise indem Federkontakte auf diese elektrischen Kontakte 122, 124 aufgebracht werden oder mittels anderer Kontaktmittel, beispielsweise elektrischer Klemmen oder ähnlichem.

Circa ein bis zwei Zentimeter von den elektrischen Kontakten 122, 124 entfernt weisen die Isolatorschichten 118, 120 Öffnungen 126, 128 auf. Dabei ist die obere Öffnung 128 in Form eines Fensters ausgestaltet, wohingegen die untere Öffnung 126 derart ausgestaltet ist, dass hier die Isolatorschicht 118 endet. Auch andere Ausgestaltungen der Öffnungen 126, 128 sind denkbar. Im Bereich dieser Öffnungen 126, 128 sind ein erstes Elektrodensystem 130 in die Öffnung 126 und ein zweites Elektrodensystem 132 in die Öffnung 128 eingebracht, derart, dass diese Elektrodensysteme 130, 132 auf den Elektrodenkontaktschichten 114, 116 aufliegen. Die Elektrodensysteme 130, 132 bilden somit in diesen Bereichen gemeinsam mit den Elektrodenkontaktschichten 114, 116 eine erste Elektrode 134 und eine zweite Elektrode 136. Dabei setzt sich das erste Elektrodensystem 130 in dem hier dargestellten Ausführungsbeispiel aus einer Ag/AgCl-Beschichtung zusammen, wohingegen das zweite Elektrodensystem 132 eine MnO₂/C(Braunstein)-Schicht ist, vermischt mit dem Enzym Glucoseoxidase (GOD).

Die erste Elektrode 134 fungiert in diesem Ausführungsbeispiel als gemeinsame Elektrode 138 und nimmt die Funktionen von Gegenelektrode 140 und Referenzelektrode 142 wahr. Die zweite Elektrode 136 wirkt in diesem Ausführungsbeispiel als Arbeitselektrode 144.

Weiterhin ist der implantierbare Sensor im Bereich der Elektroden 134, 136 mit einer Membranschicht 146 aus Polyurethan umhüllt. Diese Membranschicht 146 ist in diesem Ausführungsbeispiel undurchlässig für das Enzym Glucoseoxidase, ist jedoch zumindest teilweise durchlässig für Glucose.

Weiterhin ist an den implantierbaren Sensor 110 gemäß dem Ausführungsbeispiel in Figur 1 eine Einführspitze 148 angeformt. Diese Einführspitze 148 kann beispielsweise einstückig mit dem isolierenden Trägersubstrat 112 ausgestaltet sein oder es kann sich auch um eine separat an das isolierende Trägersubstrat 112 angebrachte Einführspitze 148 handeln.

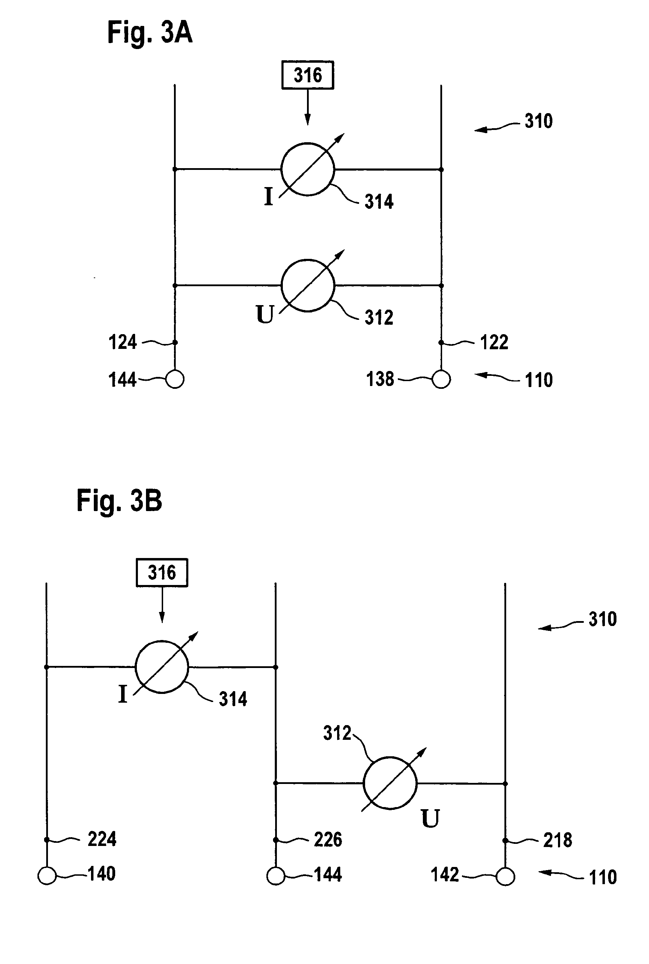

In Figur 3A ist schematisch eine Vorrichtung 310 zur Ermittlung einer Konzentration von Blutglucose unter Verwendung des Sensors 110 gemäß dem Ausführungsbeispiel in Figur 1 dargestellt. Dabei ist der Sensor 110 hier nur symbolisch durch Andeutung der Arbeitselektrode 144 und der gemeinsamen Elektrode 138 symbolisiert. Die Vorrichtung 310 weist eine Spannungsmessungsvorrichtung 312 auf, mittels derer eine elektrochemische Potenzialdifferenz (Spannung) zwischen der Arbeitselektrode 144 und der gemeinsamen Elektrode 138 gemessen werden kann. Weiterhin weist die Vorrichtung 310 eine Strommessvorrichtung 314 auf, mittels derer ein Stromfluss zwischen der Arbeitselektrode 144 und der gemeinsamen Elektrode 138 gemessen werden kann. Schließlich ist eine Regelvorrichtung 316 vorgesehen, welche den zwischen Arbeitselektrode 144 und gemeinsamer Elektrode 138 fließenden Strom derart regelt, dass die zwischen Arbeitselektrode 144 und gemeinsamer Elektrode 138 gemessene Spannung einer vorgegebenen Zollspannung entspricht. Zu diesem Zweck kann die Regelvorrichtung 316 beispielsweise eine eigene Spannungsquelle aufweisen, welche variabel ist. Aus den erforderlichen Einstellungen dieser zusätzlichen Spannungsquelle der Regelvorrichtung 316 kann dann beispielsweise auf die elektrochemische Potenzialdifferenz zwischen Arbeitselektrode 144 und gemeinsamer Elektrode 138 geschlossen werden.

Die Bestandteile der Vorrichtung 310 können räumlich getrennt voneinander sein. So kann der Sensor 110 beispielsweise teilweise oder vollständig in ein Körpergewebe implantiert werden, wohingegen die übrige Vorrichtung 310 außerhalb des Körpergewebes, beispielsweise auf der Hautoberfläche oder in einem separaten Gerät, untergebracht sind. Von der Strommessvorrichtung 314 und der Spannungsmessvorrichtung 312 führen dann entsprechende Leitungen zu den elektrischen Kontakten 122, 124 des Sensors 110.

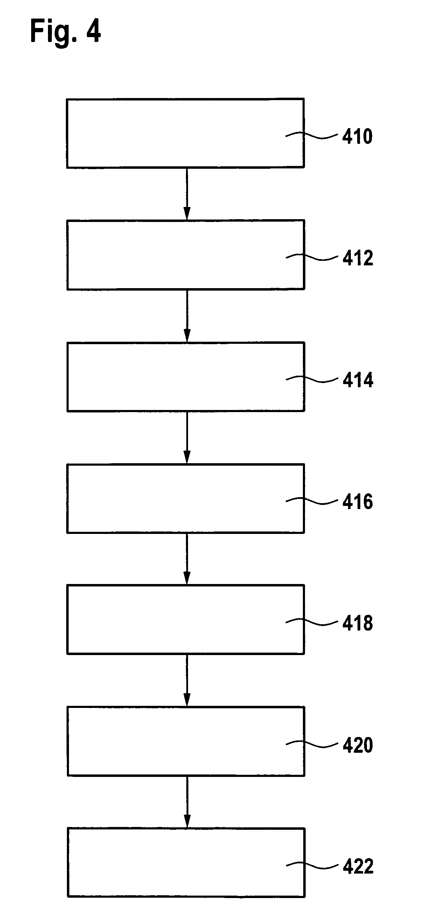

In Figur 4 ist ein Ausführungsbeispiel eines erfindungsgemäßen Verfahrens zur Herstellung eines Sensors 110 gemäß dem in Figur 1 dargestellten Ausführungsbeispiel dargestellt. Es sei darauf hingewiesen, dass jedoch auch andere Verfahren eingesetzt werden können, um den erfindungsgemäßen implantierbaren Sensor 110 gemäß der Darstellung in Figur 1 herzustellen. Auch müssen die dargestellten Verfahrensschritte nicht notwendigerweise in der angegebenen Reihenfolge durchgeführt werden.

In einem ersten Verfahrensschritt 410 werden großflächig die Elektrodenkontaktschichten 114, 116 auf eine Trägerfolie aufgebracht. Die Trägerfolie muss nicht notwendigerweise identisch sein mit den isolierenden Trägersubstraten 112, da diese isolierenden Trägersubstrate 112 aus der Trägerfolie erst durch ein späteres Schneiden hergestellt werden (siehe unten). Insofern sind sowohl Elektrodenkontaktschichten 114, 116 als auch Trägerfolie nach Durchführung dieses ersten Verfahrensschrittes 410 noch großflächig, so dass ein großflächiges "Sandwich" einer Trägerfolie, eingebettet zwischen zwei Elektrodenkontaktschichten 114, 116, entsteht. Auch die Elektrodenkontaktschichten nehmen ihre Streifenform somit erst nach dem Schneiden an. Zum Aufbringen der Elektrodenkontaktschichten 114, 116 lassen sich beispielsweise die oben beschriebenen Schichttechnologien verwenden, wobei vorzugsweise Laminiertechniken eingesetzt werden. Beispielsweise lassen sich dabei Metallfolien einsetzen, welche typischerweise Schichtdicken im Bereich von einigen 10 Mikrometern aufweisen. Es sind jedoch auch, je nach eingesetzter Schichttechnologie, dünnere oder dickere Schichten denkbar, beispielsweise Schichtdicken im Bereich von einigen 10 bis einigen 100 Nanometern bei Verwendung eines Aufdampf- oder Sputterverfahrens oder Schichtdicken im Bereich von 100 Mikrometern bei Rolle-zu-Rolle-Laminierverfahren.

In einem zweiten Verfahrensschritt, Schritt 412 in Figur 4, werden die Isolatorschichten 118, 120 aufgebracht. Dabei werden vorzugsweise selbstklebende Folien verwendet, welche wiederum großflächig auf die nach Durchführung von Schritt 410 entstandene Sandwichstruktur aufgebracht werden. Zur Erzeugung der Öffnungen 126, 128 kann beispielsweise diese selbstklebende Folie von vorneherein perforiert oder strukturiert sein, wobei jedoch eine genaue Positionierung in der Regel nicht erforderlich ist, da die genaue Positionierung der Öffnungen 126, 128 in vielen Fällen unkritisch ist. Derartige Laminierverfahren sind dem Fachmann bekannt und sind in vielfältiger Weise einsetzbar. Es lassen sich die Öffnungen 126, 128 auch nachträglich einbringen, beispielsweise durch nachträgliches Schneiden und Abziehen der Isolatorschichten 118, 120.

In einem dritten Verfahrensschritt, Schritt 414 Figur 4, wird das erste Elektrodensystem 130 aufgebracht. Dabei handelt es sich, wie oben beschrieben, um eine Ag/AgCl-Beschichtung. Zum Aufbringen dieser Ag/AgCl-Schicht kann z. B. eine Ag/AgCl-Paste, welche beispielsweise durch Vermengen von Silber und Silberchlorid-Partikeln mit einem Lösungsmittel gebildet ist, durch ein Druckverfahren (z. B. Siebdruck, Tampondruck, Schablonendruck) und/oder ein sonstiges Beschichtungsverfahren (z. B. Rakeln, Verdüsung, insbesondere durch Schlitzdüsen, Roll-Coating, o. ä.) in die Öffnung 126 eingebracht werden. Vorzugsweise wird dabei die Öffnung 126 vollständig bedeckt. Auch andere Druckverfahren lassen sich einsetzen. Es sei darauf hingewiesen, dass der in Figur 1 dargestellte Fall einen "Idealfall" darstellt, welcher in der Praxis nicht notwendigerweise in dieser Form auftreten muss. So kann beispielsweise das erste Elektrodensystem 130 auch mit der ersten Isolatorschicht 118 überlappen, was die Funktionalität der gemeinsamen Elektrode 138 nicht stört. Eine genaue Positionierung ist somit nicht erforderlich. Es kann sogar die erste Isolatorschicht 118 über einen großen Bereich mitbeschichtet werden.

Anschließend wird in Schritt 416 das zweite Elektrodensystem 132 aufgebracht. Hierbei wird, wie oben beschrieben, eine Mischung aus Braunstein und Glucoseoxidase verwendet. Dabei kann zum Aufbringen dasselbe Verfahren wie in Verfahrensschritt 414 eingesetzt werden, beispielsweise wiederum ein Druckverfahren und/oder sonstiges Beschichtungsverfahren. Auch andere Verfahren sind denkbar. Wiederum wird eine Paste eingesetzt, welche nach einer entsprechenden Trocknung fest ist und somit unlöslich ist in der umgebenden Körperflüssigkeit (Elektrolyt). Anstelle einer Mischung aus Braunstein und Glucoseoxidase kann auch zunächst eine reine Braunsteinpaste verwendet werden, auf welche dann nach Trocknung beispielsweise Glucoseoxidase aufdispensiert wird.

Anschließend wird in Verfahrensschritt 418 der bislang großflächige Schichtaufbau durch ein Präzisionsschneideverfahren hergestellt, wodurch Streifen der Breite B (vergleiche Figur 1) zu entstehen. Diese Streifen haben eine Längserstreckung parallel zu einer Einführrichtung 150 (vergleiche Figur 1). Eine genaue Positionierung beim Präzisionsschneiden senkrecht zu dieser Einführrichtung 150 ist nicht erforderlich, im Gegensatz zu herkömmlichen Verfahren, bei welchen strukturierte Elektroden in Einführrichtung 150 verwendet werden, zwischen denen die Schnitte exakt positioniert werden müssen.

Anschließend wird in Verfahrensschritt 420 die Einführspitze 148 an die nunmehr entstandenen isolierenden Trägersubstrate 112 angeformt. Beispielsweise können Einführspitzen 148 durch gleichzeitiges Schmelzen und Ziehen erzeugt werden, oder es kann auch eine entsprechende Heißformung erfolgen. Auch können alternativ oder zusätzlich separate Spitzen an die isolierenden Trägersubstrate 112 angeformt werden, beispielsweise durch Verschmelzen mit den isolierenden Trägersubstraten 112. Verschiedene Möglichkeiten sind denkbar. Alternativ kann dieser Verfahrensschritt jedoch auch weggelassen werden, da, wie oben erwähnt, der implantierbare Sensor 110 auch beispielsweise in eine separate Einführnadel eingefügt werden kann. Bevorzugt ist jedoch, wenn der Sensor 110 über eine eigene Einführspitze 148 verfügt, so dass die Elektroden 134, 136 frei von der Körperflüssigkeit (Elektrolyt) umspült werden.

Anschließend wird im letzten Verfahrensschritt, 422 in Figur 4, die Membranschicht 146 auf den Sensor 110 aufgebracht. Dabei kann beispielsweise ein einfaches Tauchverfahren verwendet werden, bei welchem der Sensor 110 (ohne Membranschicht) in eine Lösung oder eine andere das Membranmaterial (oder ein Vorprodukt desselben) enthaltende Flüssigkeit eingetaucht wird. Nach dem Tauchen kann optional zusätzlich durch Spincoating ein gleichmäßiger Flüssigkeitsfilm erzeugt werden, der anschließend getrocknet werden kann. Anschließend wird ein Trocknungsschritt durchgeführt, bei welchem die Membranschicht 146 trocknet. Eine genaue Positionierung des Tauchvorganges ist nicht erforderlich, da lediglich die Elektroden 134, 136 bedeckt werden müssen und eine darüber hinausgehende Bedeckung des Sensors 110 unerheblich für die Messergebnisse ist. Wie oben beschrieben, lassen sich beispielsweise Polyurethane als Materialien für die Membranschicht 146 einsetzen. Es lassen sich auch andere Verfahren zum Aufbringen einsetzen, beispielsweise Verfahren, bei welchen eine Polymerisierung erst nach dem Eintauchen und beim Trocknen erfolgt, oder Verfahren wie Sprühverfahren oder Druckverfahren. Als Membranmaterialien sollten insbesondere biokompatible Materialien eingesetzt werden, das heißt Materialien, welche während der Messdauer (typischerweise eine Woche, zum Teil auch mehr, zuzüglich einer "Sicherheitszeit") keine Reaktionen mit dem umgebenden Körpergewebe und/oder der Körperflüssigkeit durchführen oder toxische Substanzen in nennenswertem Maße abgeben.

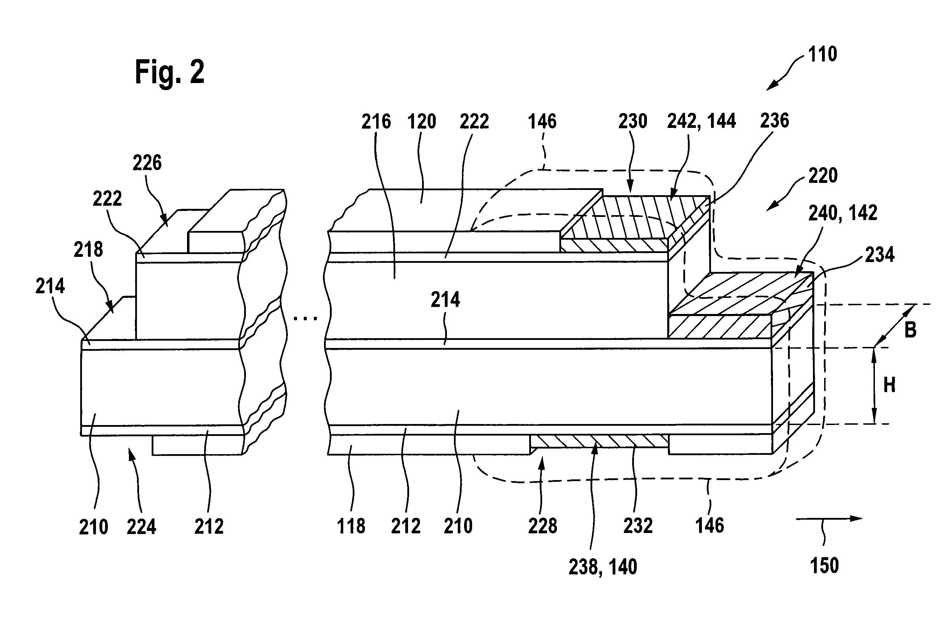

In Figur 2 ist ein zweites Ausführungsbeispiel eines erfindungsgemäßen implantierbaren Sensors 110 dargestellt, wobei in diesem Ausführungsbeispiel die Einführspitze 148 nicht dargestellt oder weggelassen ist. Der Sensor 110 gemäß dem Ausführungsbeispiel in Figur 2 weist ein erstes isolierendes Trägersubstrat 210 auf, welche zwischen einer ersten Elektrodenkontaktschicht 212 und einer zweiten Elektrodenkontaktschicht 214 eingebettet ist. An die zweite Elektrodenkontaktschicht 214 schließt sich ein zweites isolierendes Trägersubstrat 216 an, welches sich jedoch nicht über die gesamte Längserstreckung des ersten isolierenden Trägersubstrats 210 erstreckt. So ist am linken Ende des Sensors 110 (das heißt entgegengesetzt der Einführrichtung 150) ein Bereich der zweiten Elektrodenkontaktschicht 214 unbedeckt belassen, so dass sich dort ein elektrischer Kontakt zu Kontaktierung der zweiten Elektrodenkontaktschicht 214 ausbildet (zur Kontaktierung: siehe oben). Am (in Einführrichtung 150) rechten Ende endet das zweite isolierende Trägersubstrat 216 vor dem ersten isolierenden Trägersubstrat 210, so dass sich in diesem Bereich eine Stufe 220 ausbildet. Es sei hier darauf hingewiesen, dass alternativ diese Stufe 220 anstatt "nach oben" wie in Figur 2 auch "nach unten" ausgebildet sein kann, so dass insgesamt eine Invertierung des Schichtaufbaus möglich ist.

Auf der dem ersten isolierenden Trägersubstrat 210 abgewandten Seite ist das zweite isolierende Trägersubstrat 216 mit einer dritten Elektrodenkontaktschicht 222 beschichtet. Wiederum erstrecken sich sämtliche Elektrodenkontaktschichten 212, 214, 222 über die gesamte Breite B des Sensors 110. Als Materialen für die Elektrodenkontaktschichten 212, 214, 222 können grundsätzlich dieselben Materialien eingesetzt werden wie im Fall von Figur 1.

Wie auch in Figur 1 ist auch der Sensor 110 gemäß Figur 2 außenseitig mit Isolatorschichten 118, 120 beschichtet, welche in diesem Fall die erste Elektrodenkontaktschicht 212 und die dritte Elektrodenkontaktschicht 222 außenseitig elektrisch gegenüber dem umgebenden Elektrolyten, insbesondere der Körperflüssigkeit, isolieren. Wiederum kann es sich dabei um selbstklebende Folien handeln. Wie auch in Figur 1 enden auch im Beispiel gemäß Figur 2 die Isolatoren 118, 120 am linken Ende des Sensors 110 vor dem Ende der zugehörigen isolierenden Trägersubstrate 210 beziehungsweise 216 dergestalt, dass elektrische Kontakte 224, 226 frei bleiben, über welche die Elektrodenkontaktschichten 212 beziehungsweise 222 elektrisch kontaktiert werden können.

Analog zur Ausführung in Figur 1 sind auch im Ausführungsbeispiel gemäß Figur 2 Öffnungen 228, 230 in den Isolatorschichten 118 und 120 vorgesehen. Diese Öffnungen 228, 230, welche beispielsweise wiederum als "Fenster" und/oder als einfache freibleibende Bereiche der Elektrodenkontaktschichten 212, 222 ausgebildet sein können, können wiederum bereits beim Aufbringen der Isolatorschichten 118, 120 erzeugt werden oder können durch späteres Strukturieren erzeugt werden.

In die Öffnung 228, auf die zweite Elektrodenkontaktschicht 214 im Bereich der Stufe 220 und in die Öffnung 230 werden ein erstes Elektrodensystem 232, ein zweites Elektrodensystem 234 und ein drittes Elektrodensystem 236 ein- beziehungsweise aufgebracht. Dabei wird als erstes Elektrodensystem 232 und als zweites Elektrodensystem 234 wiederum, wie auch im Ausführungsbeispiel gemäß Figur 1, eines Ag/AgCl-Schicht verwendet. Als drittes Elektrodensystem 232 wird wiederum eine Braunstein-GOD-Schicht verwendet, analog zum zweiten Elektrodensystem 132 in Figur 1. Gemeinsam mit den zugehörigen Elektrodenkontaktschichten 212, 214 bzw. 222 bilden diese Elektrodensysteme 232, 234, 236 dann jeweils eine erste Elektrode 238, eine zweite Elektrode 240 und eine dritte Elektrode 242. Die erste Elektrode 238 wirkt in diesem Ausführungsbeispiel als Gegenelektrode 140, die zweite Elektrode 240 als Referenzelektrode 142 und die dritte Elektrode 242 als Arbeitselektrode 144. Somit sind in diesem Ausführungsbeispiel gemäß Figur 2 die drei Elektroden 238, 240, 242 allesamt in unterschiedlichen Schichtebenen des Schichtaufbaus angeordnet. Somit lassen sich diese Elektroden sehr breit (das heißt in diesem Fall über die volle Breite B) ausgestalten, sind jedoch trotzdem zuverlässig voneinander getrennt.

Weiterhin ist der Sensor 110 im Bereich der Elektroden 238, 240, 242 noch, analog zu Figur 1, von einer Membranschicht 146 umhüllt, welche analog zum Ausführungsbeispiel in Figur 1 ausgestaltet sein kann.

Bezüglich des Schichtdickenverhältnisses und des Aspektverhältnisses k=H/B sei in diesem Fall darauf hingewiesen, dass die oben genannte Bedingung nicht notwendig für die Höhe eines einzelnen isolierenden Trägersubstrats 210, 216 gelten soll, sondern vorzugsweise für die gesamte Dicke des in Figur 2 dargestellten Schichtaufbaus. Dies ergibt sich daraus, dass, um eine möglichst abknickfreie Einführung des Sensors 110 unter die Haut eines Patienten zu gewährleisten, der Sensor 110 insgesamt einen näherungsweise quadratischen Querschnitt haben sollte.

In Figur 3B ist eine Vorrichtung 310 zur Bestimmung einer Blutglucosekonzentration unter Verwendung eines Sensors 110 gemäß dem in Figur 1 dargestellten Ausführungsbeispiel dargestellt. Im Gegensatz zur Vorrichtung gemäß Figur 3A sind nunmehr die drei Elektroden 140, 144, 142 als getrennte Elektroden ausgestaltet. Alle drei Elektroden werden wiederum von dem Elektrolyten der Körperflüssigkeit umspült. An der Arbeitselektrode 144, welche wiederum Glucoseoxidase aufweist, findet wiederum eine Umwandlung von Glucose in Gluconolacton unter Bildung von Elektronen satt. Das elektrochemische Potenzial der Arbeitselektrode 144 wird also wiederum durch die Konzentration der Glucose in der Körperflüssigkeit bestimmt.

Wiederum weist die Vorrichtung 310 eine Spannungsmessungsvornchtung 312 zur Spannung zwischen Arbeitselektrode 144 und Referenzelektrode 142 auf sowie eine Strommessvorrichtung 314 zur Messung eines zwischen der Gegenelektrode 140 und der Arbeitselektrode 144 fließenden Stroms. Weiterhin ist wiederum eine Regelvorrichtung 316 vorgesehen, welche den zwischen Gegenelektrode 140 und Arbeitselektrode 144 fließenden Strom derart regelt, dass die Spannung zwischen Arbeitselektrode 144 und Referenzelektrode 142 einen vorgegebenen Sollwert erreicht. Im Übrigen funktioniert das Verfahren wie anhand von Figur 3A beschrieben.

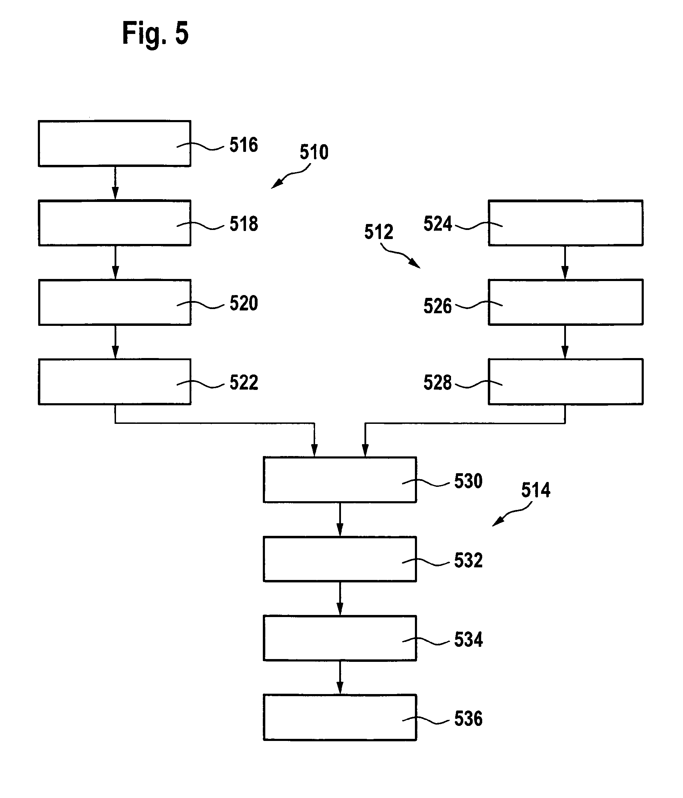

In Figur 5 ist schließlich ein erfindungsgemäßes Ausführungsbeispiel eines Herstellungsverfahrens zur Herstellung eines Sensors 110 gemäß dem Ausführungsbeispiel in Figur 2 dargestellt. Es sei jedoch wiederum darauf hingewiesen, dass auch andere Herstellungsverfahren zur Herstellung dieses Sensors 110 einsetzbar sind. Auch können wiederum die Verfahrensschritte in einer anderen Rheinfolge durchgeführt werden, und es können wiederum auch zusätzliche, nicht dargestellte Verfahrensschritte durchgeführt werden.

Anstelle eines schichtweisen Aufbaus "von unten nach oben" ist das Verfahren in Figur 5 in zwei Teilverfahren aufgeteilt, bei denen ein erster Teilschichtaufbau (Teilverfahren 510) und ein zweiter Teilschichtaufbau (Teilverfahren 512) unabhängig voneinander hergestellt werden. Anschließend werden im gemeinsamen Verfahren 514 die beiden Teilschichtaufbauten zusammengefügt und weiterverarbeitet.

Im Teilverfahren 1 wird zunächst, analog zu Verfahrensschritt 410 in Figur 4, in Schritt 516 eine erste Trägerfolie großflächig mit den Elektrodenkontaktschichten 212, 214 beschichtet. Anschließend wird auf einer Seite des so entstandenen Sandwichaufbaus in Schritt 518 die Isolatorfolie 118 auf die erste Elektrodenkontaktschicht 212 aufgebracht, analog zu Verfahrensschritt 412 gemäß Figur 4, jedoch nur einseitig.

Anschließend wird in Schritt 520 das erste Elektrodensystem 232 in die Öffnung 228 in der Isolatorschicht 118 eingebracht, beispielsweise mittels des anhand von Figur 4 beschriebenen Verfahrens.

Weiterhin wird in Schritt 522 das zweite Elektrodensystem 234 auf einen Bereich der zweiten Elektrodenkontaktschicht 214 aufgebracht, in welchem später die Stufe 220 ausgebildet ist. Alternativ kann das zweite Elektrodensystem 234 auch beispielsweise erst im Rahmen des gemeinsamen Verfahrens 514 aufgebracht werden, also nach Zusammenfügen der beiden Teilschichtaufbauten (siehe unten).

Nach Durchführung der Verfahrensschritte 516 bis 522 ist der erste Teilschichtaufbau fertig. In den Verfahrensschritten 524 bis 528 wird (insbesondere unabhängig zu den obigen Verfahrensschritten, also beispielsweise parallel) ein zweiter Teilschichtaufbau hergestellt. Dazu wird zunächst in Verfahrensschritt 524 eine zweite Trägerfolie großflächig und einseitig mit der dritten Elektrodenkontaktschicht 222 beschichtet. Anschließend wird in Verfahrensschritt 526 die Isolatorschicht 120 auf die dritte Elektrodenkontaktschicht 222 aufgebracht, wobei eine Öffnung 230 verbleibt. Für die Techniken und Materialien der Aufbringung der einzelnen Schichten sei wiederum auf das Ausführungsbeispiel gemäß Figur 4 verwiesen.

Anschließend wird im letzten Verfahrensschritt 528 des Teilverfahrens 512 das dritte Elektrodensystem 236 in die Öffnung 230 eingebracht. Damit ist das zweite Teilverfahren 512 fertig, und der zweite Teilschichtaufbau ist hergestellt.

Anschließend wird im gemeinsamen Verfahren 514 im Verfahrensschritt 530 der zweite Teilschichtaufbau, welcher sich aus der zweiten Trägerfolie, der dritten Elektrodenkontaktschicht 222, der Isolatorschicht 120 und dem dritten Elektrodensystem 236 zusammensetzt, auf den ersten Teilschichtaufbau, welcher sich aus die Isolatorschicht 118, der ersten Elektrodenkontaktschicht 212, der ersten Trägerfolie, der zweiten Elektrodenkontaktschicht 214 und dem ersten Elektrodensystem 232 und dem zweiten Elektrodensystem 234 zusammensetzt, aufgebracht. Beispielsweise können für dieses Aufbringen wiederum Laminiertechniken verwendet werden.

Im anschließenden Verfahrensschritt 532 findet wiederum ein Präzisionsschneiden statt, bei welchem die bislang großflächigen Schichtaufbauten in streifenförmige Sensoren 110 geschnitten werden. Wiederum ist hierbei, wie oben beschrieben, eine exakte Positionierung nicht unbedingt erforderlich.

Anschließend wird in Verfahrensschritt 534 optional eine Einführspitze 148 an den Schichtaufbau angeformt, welche in der Darstellung gemäß Figur 2 nicht abgebildet ist. Schließlich wird im letzten Verfahrensschritt, Schritt 536, die Membranschicht 146 aufgebracht, analog zu Verfahrensschritt 422, beispielsweise wiederum durch ein Tauchverfahren.

Die in den Figuren 4 und 5 dargestellten beispielhaften Verfahren zur Herstellung der Sensoren 110 lassen sich gut großtechnisch realisieren. So lassen sich insbesondere Rolle-zu-Rolle-Verfahren einsetzen, für welche aus anderen Bereichen der Technik Automaten zur Verfügung stehen, so dass keine Spezialanfertigungen aufwändiger Herstellungsgeräte erforderlich ist. Das Verfahren ist äußerst kostengünstig und kann mit hoher Ausbeute und hohem Durchsatz gefahren werden.

### Bezugszeichenliste

- 110: implantierbarer Sensor
- 112: isolierendes Trägersubstrat
- 114: erste Elektrodenkontaktschicht
- 116: zweite Elektrodenkontaktschicht
- 118: Isolatorschicht
- 120: Isolatorschicht
- 122: elektrischer Kontakt
- 124: elektrischer Kontakt
- 126: Öffnung des Isolatorschicht 118
- 128: Öffnung der Isolatorschicht 120
- 130: erstes Elektrodensystem
- 132: zweites Elektrodensystem
- 134: erste Elektrode
- 136: zweite Elektrode
- 138 8: gemeinsame Elektrode
- 140: Gegenelektrode
- 142: Referenzelektrode
- 144: Arbeitselektrode
- 146: Membranschicht
- 148: Einführspitze
- 150: Einführrichtung
- 210: erstes isolierendes Trägersubstrat
- 212: erste Elektrodenkontaktschicht
- 214: zweite Elektrodenkontaktschicht
- 216: zweites isolierendes Träger-Substrat
- 218: elektrischer Kontakt
- 220: Stufe
- 222: dritte Elektrodenkontaktschicht
- 224: elektrischer Kontakt
- 226: elektrischer Kontakt
- 228: Öffnung
- 230: Öffnung
- 232: erstes Elektrodensystem
- 234: zweites Elektrodensystem
- 236: drittes Elektrodensystem
- 238: erste Elektrode
- 240: zweite Elektrode
- 242: dritte Elektrode
- 310: Vorrichtung zur Ermittlung einer Blutglucosekonzentration
- 312: Spannungsmessungsvornchtung
- 314: Strommessvorrichtung
- 316: Regelvorrichtung
- 410: Aufbringen der Elektrodenkontaktschichten
- 412: Aufbringen Isolatorschichten
- 414: Aufbringen erstes Elektrodensystem
- 416: Aufbringen zweites Elektrodensystem
- 418: Präzisionsschneideverfahren
- 420: Anformung Einführspitze
- 422: Aufbringen Membranschicht
- 510: Teilverfahren 1: Herstellung erster Teilschichtaufbau
- 512: Teilverfahren 2: Herstellung zweiter Teilschichtaufbau
- 514: gemeinsames Verfahren
- 516: Aufbringen erste und zweite Elektrodenkontaktschicht auf erste Trägerfolie
- 518: Aufbringen Isolatorfolie
- 520: Aufbringen erstes Elektrodensystem
- 522: Aufbringen zweites Elektrodensystem
- 524: Aufbringen dritte Elektrodenkontaktschicht auf zweite Trägerfolie
- 526: Aufbringen Isolatorschicht
- 528: Aufbringen drittes Elektrodensystem
- 530: Aufbringen zweiter Teilschichtaufbau auf ersten Teilschichtaufbau
- 532: Präzisionsschneiden
- 534: Anformen Einführspitze
- 536: Aufbringen Membranschicht

## Patentansprüche

1. Implantierbarer Sensor (110) zur Ermittlung einer Konzentration mindestens eines Analyten in einem Medium, insbesondere einem Körpergewebe und/oder einer Körperflüssigkeit, wobei der implantierbare Sensor (110) einen Schichtaufbau aufweist, wobei der Schichtaufbau mindestens ein isolierendes Trägersubstrat (112; 210, 216) und mindestens zwei Elektroden (134, 136; 238, 240, 242) aufweist, wobei die mindestens zwei Elektroden (134, 136; 238, 240, 242) mindestens eine Arbeitselektrode (144) und mindestens eine weitere Elektrode (134, 136; 238, 240, 242), insbesondere mindestens eine Gegenelektrode (140) und/oder mindestens eine Referenzelektrode (142), umfassen, **dadurch gekennzeichnet, dass** das mindestens eine isolierende Trägersubstrat (112; 210, 216) mindestens ein elektrisch nichtleitendes Material umfasst und dass die Arbeitselektrode (144) und die mindestens eine weitere Elektrode (134, 136; 238, 240, 242) in unterschiedlichen Schichtebenen des Schichtaufbaus angeordnet sind.

2. Implantierbarer Sensor (110) gemäß dem vorhergehenden Anspruch, **gekennzeichnet** weiterhin durch mindestens zwei die mindestens zwei Elektroden (134, 136; 238, 240, 242) elektrisch kontaktierende Elektrodenkontaktschichten (114, 116; 212, 214; 222).

3. Implantierbarer Sensor (110) gemäß dem vorhergehenden Anspruch, **dadurch gekennzeichnet, dass** das mindestens eine isolierende Trägersubstrat (112) eine Breite aufweist, wobei sich die mindestens zwei Elektroden (134, 136; 238, 240, 242) und/oder die mindestens zwei Elektrodenkontaktschichten (114, 116; 212, 214; 222) im Wesentlichen über die gesamte Breite des mindestens einen isolierenden Trägersubstrats (112) erstrecken.

4. Implantierbarer Sensor (110) gemäß einem der beiden vorhergehenden Ansprüche, **dadurch gekennzeichnet, dass** die mindestens zwei Elektrodenkontaktschichten (114, 116; 212, 214; 222) zumindest teilweise, vorzugsweise vollständig, durch mindestens zwei Isolatorschichten (118, 120), vorzugsweise durch selbstklebende Folienschichten, gegen das Medium, insbesondere das Körpergewebe und/oder die Körperflüssigkeit, abgedeckt, vorzugsweise elektrisch isoliert sind.

5. Implantierbarer Sensor (110) gemäß einem der vorhergehenden Ansprüche, **gekennzeichnet durch** mindestens eine den Schichtaufbau, insbesondere die mindestens zwei Elektroden (134, 136; 238, 240, 242), ganz oder teilweise umschließende Membranschicht (146), insbesondere eine biokompatible Membranschicht (146).

6. Implantierbarer Sensor (110) gemäß dem vorhergehenden Anspruch, **dadurch gekennzeichnet, dass** die mindestens eine Membranschicht (146) eine zumindest teilweise Durchlässigkeit für den mindestens einen Analyten aufweist.

7. Implantierbarer Sensor (110) gemäß dem vorhergehenden Anspruch, **dadurch gekennzeichnet, dass** die mindestens eine Membranschicht (146) ein Polyurethan aufweist.

8. Implantierbarer Sensor (110) gemäß einem der vorhergehenden Ansprüche, **dadurch gekennzeichnet, dass** das mindestens eine isolierende Trägersubstrat (112) als Werkstoff ein Polymer, insbesondere ein isolierendes Polymer, insbesondere einen Polyester, aufweist.

9. Implantierbarer Sensor (110) gemäß einem der vorhergehenden Ansprüche, **dadurch gekennzeichnet, dass** die mindestens eine Gegenelektrode (140) und die mindestens eine Referenzelektrode (142) als eine gemeinsame Elektrode (138) ausgebildet sind.

10. Implantierbarer Sensor (110) gemäß einem der vorhergehenden Ansprüche, **gekennzeichnet durch** eine Einführspitze (148) zum Einstechen des implantierbaren Sensors (110) in das Medium, insbesondere in ein Fettgewebe.

11. Implantierbarer Sensor (110) gemäß dem vorhergehenden Anspruch, **dadurch gekennzeichnet, dass** das mindestens eine isolierende Trägersubstrat (112) und/oder der implantierbare Sensor (110) eine Breite B und eine Höhe H aufweist, wobei das Verhältnis H/B oder dessen Kehrwert ein Aspektverhältnis k definiert, wobei das Aspektverhältnis k mindestens 0,3, vorzugsweise mindestens 0,5 und besonders bevorzugt mindestens 0,9 beträgt.

12. Implantierbarer Sensor (110) gemäß einem der vorhergehenden Ansprüche, **dadurch gekennzeichnet, dass** der Schichtaufbau ein gestufter Schichtaufbau ist, wobei mindestens zwei isolierende Trägersubstrat (112) vorhanden sind, wobei mindestens zwei der isolierenden Trägersubstrate (112) eine Stufe (220) bilden.

13. Implantierbarer Sensor (110) gemäß dem vorhergehenden Anspruch, **dadurch gekennzeichnet, dass** mindestens drei Elektroden (238, 240, 242) in drei verschiedenen Schichtebenen vorgesehen sind, wobei vorzugsweise mindestens eine Elektrode (240) in der Ebene der Stufe (220) vorgesehen ist.

14. Implantierbarer Sensor (110) gemäß einem der vorhergehenden Ansprüche, aufweisend mindestens einen der folgenden Schichtaufbauten:
- ein zwischen einer ersten Elektrodenkontaktschicht (114) und einer zweiten Elektrodenkontaktschicht (116) eingebettetes isolierendes Trägersubstrat (112), wobei auf der dem isolierenden Trägersubstrat (112) abgewandten Seite der ersten Elektrodenkontaktschicht (114) mindestens eine erste Elektrode (134) aufgebracht ist und wobei auf der dem isolierenden Trägersubstrat (112) abgewandten Seite der zweiten Elektrodenkontaktschicht (116) mindestens eine zweite Elektrode (136) aufgebracht ist;
- ein zwischen einer ersten Elektrodenkontaktschicht (212) und einer zweiten Elektrodenkontaktschicht (214) eingebettetes erstes isolierendes Trägersubstrat (210), ein auf die dem ersten isolierenden Trägersubstrat (112) abgewandten Seite der zweiten Elektrodenkontaktschicht (214) aufgebrachtes zweites isolierendes Trägersubstrat (216), wobei das zweite isolierende Trägersubstrat (216) in einer Längserstreckungsrichtung des implantierbaren Sensors (110) kürzer ist als das erste isolierende Trägersubstrat (210), wodurch eine Stufe (220) ausgebildet ist, und eine auf der dem ersten isolierenden Trägersubstrat (210) abgewandten Seite des zweiten isolierenden Trägersubstrats (216) aufgebrachte dritte Elektrodenkontaktschicht (222), wobei auf der dem zweiten isolierenden Trägersubstrat (216) abgewandten Seite des ersten isolierenden Trägersubstrats (210) eine erste Elektrode (138) auf der ersten Elektrodenkontaktschicht (212) aufgebracht ist, wobei im Bereich der Stufe (220) eine zweite Elektrode (240) auf die zweite Elektrodenkontaktschicht (214) aufgebracht ist und wobei auf der dem ersten isolierenden Trägersubstrat (210) abgewandten Seite des zweiten isolierenden Trägersubstrats (216) eine dritte Elektrode (242) auf die dritte Elektrodenkontaktschicht (222) aufgebracht ist.

15. Vorrichtung (310) zur Ermittlung einer Konzentration mindestens eines Analyten in einem Medium, insbesondere einem Körpergewebe und/oder einer Körperflüssigkeit, umfassend mindestens einen implantierbaren Sensor (110) gemäß einem der vorhergehenden Ansprüche sowie weiterhin mindestens eine Spannungsmessvorrichtung (312) zur Messung einer Spannung zwischen der mindestens einen Arbeitselektrode (144) und der mindestens einen Referenzelektrode (142).

16. Vorrichtung (310) gemäß dem vorhergehenden Anspruch, umfassend weiterhin mindestens eine Strommessvorrichtung (314) zur Messung eines Stroms zwischen der mindestens einen Gegenelektrode (140) und der mindestens einen Arbeitselektrode (144).

17. Verwendung eines implantierbaren Sensors (110) und/oder einer Vorrichtung (310) gemäß einem der vorhergehenden Ansprüche für eine kontinuierliche Ermittlung einer Konzentration mindestens eines Analyten in einem Medium, insbesondere einem Körpergewebe und/oder einer Körperflüssigkeit.

18. Verfahren zur Herstellung eines implantierbaren Sensors (110) zur Ermittlung einer Analytkonzentration in einem Medium, insbesondere einem Körpergewebe und/oder einer Körperflüssigkeit, **gekennzeichnet durch** folgende Schritte:
- Ein Schichtaufbau wird erzeugt, wobei folgende Verfahrensschritte eingesetzt werden:
o Mindestens zwei Elektrodenkontaktschichten (114, 116; 212, 214; 222), insbesondere mindestens zwei Metallschichten, werden großflächig in mindestens zwei verschiedenen Schichtebenen auf mindestens eine mindestens ein elektrisch nichtleitendes Material umfassende Trägerfolie aufgebracht;
o mindestens zwei Elektroden (134, 136; 238, 240, 242) werden auf die mindestens zwei Elektrodenkontaktschichten (114, 116; 212, 214; 222) aufgebracht; und
- mittels eines Präzisions-Schneideverfahrens wird der Schichtaufbau in Sensorstreifen geschnitten.

19. Verfahren gemäß dem vorhergehenden Anspruch, **dadurch gekennzeichnet, dass** mindestens eines der folgenden Verfahren eingesetzt wird:
- ein Laminierverfahren;
- ein Rolle-zu-Rolle-Folienverfahren;
- ein PVD-Verfahren und/oder ein CVD-Verfahren;
- ein nasschemisches Beschichtungsverfahren.

20. Verfahren gemäß einem der beiden vorhergehenden Ansprüche, **dadurch gekennzeichnet, dass** kein zusätzlicher Strukturierungsschritt der mindestens zwei Elektrodenkontaktschichten (114, 116; 212, 214; 222) erfolgt.

21. Verfahren gemäß einem der vorhergehenden Verfahrensansprüche, **dadurch gekennzeichnet, dass** mindestens eine erste Trägerfolie beidseitig mit Elektrodenkontaktschichten (212, 214) umgeben, insbesondere beschichtet, wird, wobei auf eine der beiden Elektrodenkontaktschichten (212) auf der der Trägerfolie abgewandten Seite mindestens eine erste Elektrode (238) aufgebracht wird und wobei auf die andere der beiden Elektrodenschichten (214) auf der der Trägerfolie abgewandten Seite mindestens eine zweite Elektrode (240) aufgebracht wird.

22. Verfahren gemäß dem vorhergehenden Anspruch, wobei zusätzlich eine zweite Trägerfolie einseitig mit einer dritten Elektrodenkontaktschicht (222) in Kontakt gebracht, insbesondere beschichtet, wird, wobei auf die dritte Elektrodenkontaktschicht (222) eine dritte Elektrode (242) aufgebracht wird, wobei die zweite Trägerfolie mit der der dritten Elektrodenkontaktschicht (222) abgewandten Seite auf die beidseitig mit Elektrodenkontaktschichten (212, 214) umgebene erste Trägerfolie aufgebracht wird, dergestalt, dass mindestens eine Stufe (220) entsteht.

23. Verfahren gemäß einem der vorhergehenden Verfahrensansprüche, **dadurch gekennzeichnet, dass** zusätzlich mindestens eine Isolatorschicht (118, 120), insbesondere mindestens eine selbstklebende Folienschicht, aufgebracht wird.
